# EUROPEAN PATENT SPECIFICATION

(11) **EP 3 914 318 B1**
(45) Date of publication and mention of the grant of the patent: **17.01.2024**
(21) Application number: 20715189.5
(22) Date of filing: 22.01.2020
(51) Int. Cl.: A61M 1/36

(54) **APPLIANCE FOR THE SUPPORT OF BIOMEDICAL DEVICES DURING EXTRACORPOREAL CIRCULATION**
GERÄT ZUR UNTERSTÜTZUNG BIOMEDIZINISCHER VORRICHTUNGEN WÄHREND DER EXTRAKORPORALEN ZIRKULATION
APPAREIL POUR LE SUPPORT DE DISPOSITIFS BIOMÉDICAUX PENDANT UNE CIRCULATION EXTRACORPORELLE

(30) Priority: 25.01.2019 IT 201900001149
(43) Date of publication of application: 01.12.2021
(73) Proprietor: Eurosets S.r.l., 41036 Medolla (MO) (IT)
(72) Inventor: PETRALIA, Antonio, 41036 Medolla (MO) (IT); GHELLI, Nicola, 41036 Medolla (MO) (IT); FONTANILI, Paolo, 41036 Medolla (MO) (IT); ZERBINI, Alessandro, 41036 Medolla (MO) (IT)
(74) Representative: Zoli, Filippo
(86) International application number: PCT/IB2020/050477
(87) International publication number: WO 2020/152589

(56) References cited:
- US-A1- 2003 163 078
- US-A1- 2005 063 860
- US-A1- 2011 208 108
- US-B2- 9 033 298

## Description

### Technical Field

The present invention relates to an appliance for the support of biomedical devices during extracorporeal circulation, in particular for the support of an oxygenator and of the relevant pumping assembly.

### Background Art

As is well known, in the event of certain surgical operations, during which the functions of the patient's heart are temporarily suspended, extracorporeal blood circuits are created using the so-called "heart-lung" machines.

The heart-lung machines comprise a series of devices including a filtration device (also called "venous reserve") adapted to filter the blood coming from the patient, a heat exchanger adapted to regulate the temperature of the blood coming out of the filtration device, and an oxygenator adapted to provide the correct supply of oxygen to the blood intended to be returned to the patient. In particular, the blood coming from the patient is sent to the oxygenator by means of a relevant pumping assembly, which generally comprises a centrifugal pump of the dragging or magnetic levitation type, i.e. provided with a rotor element adapted to send the incoming blood to the oxygenator as a result of the rotation thereof and with a stator element adapted to drive the rotor element in rotation. During extracorporeal circulation, a number of parameters have to be monitored and controlled, including the number of revolutions of the rotor element and the flow rate of blood sent to the oxygenator.

Appropriately, during the extracorporeal circulation applied to a patient, a supporting appliance is generally used having a load-bearing body provided with first supporting means of the oxygenator, second supporting means of the relevant pumping assembly and provided with a command and control unit connectable at least to the pumping assembly for the command and control of the aforementioned parameters.

This appliance is now particularly used also in non-hospital environments since extracorporeal circulation is also applied in emergency situations, e.g. road rescue, where there is the need to adequately support the various devices in a dynamic context and with a flexible manner.

The appliances of known type for the support of biomedical devices, in particular for the support of the oxygenator and the relevant pumping assembly, do have some drawbacks.

In particular, they do not allow the oxygenator and the relevant pumping assembly to be easily and practically supported and moved according to the specific needs.

Another drawback of these appliances of known type is that they are not able to adequately support the oxygenator and the relevant pumping assembly during extracorporeal circulation activity both in non-hospital emergency situations and in hospital situations.

Appliances of the know type for the supporting of biomedical devices are disclosed by US 2011/208108A1, US 2003/163078A1, US 2005/063860 A1 and US 9033298B2.

### Description of the Invention

The main aim of the present invention is to devise an appliance which allows supporting the oxygenator and the relevant pumping assembly in a practical and functional manner during the application of extracorporeal circulation to a patient.

Within this aim, one object of the present invention is to enable medical staff to set up the oxygenator and the relevant pumping assembly in the optimal position with respect to the patient, so as to facilitate the connection to the other medical devices and the fluid dynamics of the blood.

Another object is to simplify the movement of the oxygenator and of the relevant pumping assembly in the event of their position needing to be changed with respect to the load-bearing body.

Yet another object of the present invention is to devise an appliance that is flexible in use and that allows effectively supporting the oxygenator and the relevant pumping assembly, or even just one of them so as to allow the separate movement thereof, even if the patient has to be moved, without interrupting the extracorporeal circulation applied to them.

Another object of the present invention is to devise an appliance for the support of biomedical devices during the extracorporeal circulation which allows overcoming the aforementioned drawbacks of the prior art in the ambit of a simple, rational, easy, effective to use and low cost solution.

The objects set out above are achieved by the present appliance for the support of biomedical devices during extracorporeal circulation according to claim 1.

### Brief Description of the Drawings

Other characteristics and advantages of the present invention will be more evident from the description of a preferred, but not exclusive, embodiment of an appliance for the support of biomedical devices during extracorporeal circulation, illustrated as an indication, but not limited to, in the attached tables of drawings in which:

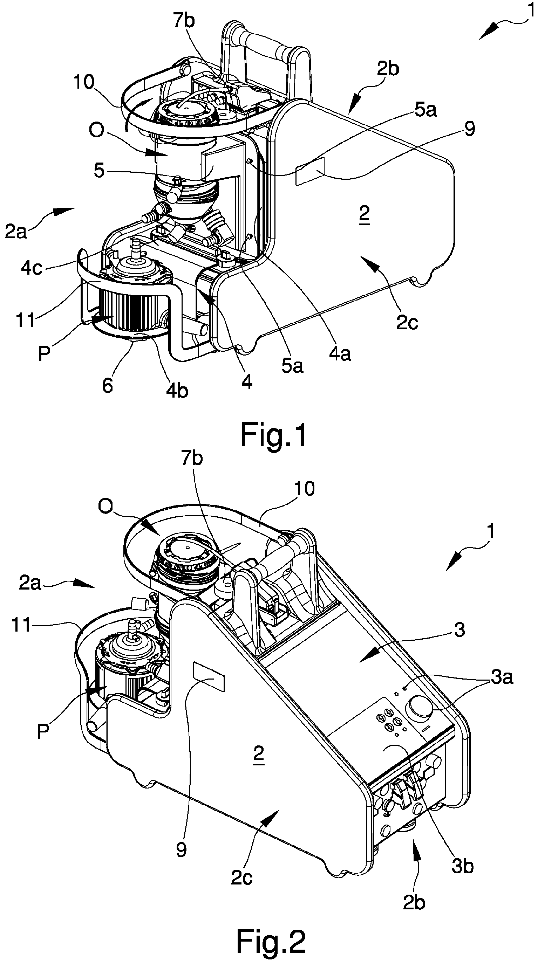

Figure 1 is a first axonometric view of an appliance according to the invention in a first configuration of use;
Figure 2 is a second axonometric view of the appliance in Figure 1;

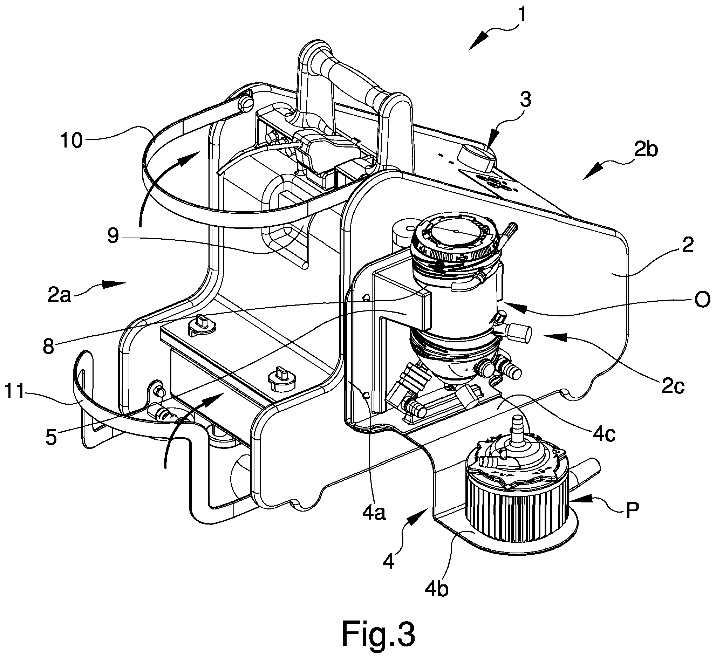

Figure 3 is an axonometric view of the appliance in Figure 1 in a second configuration of use;

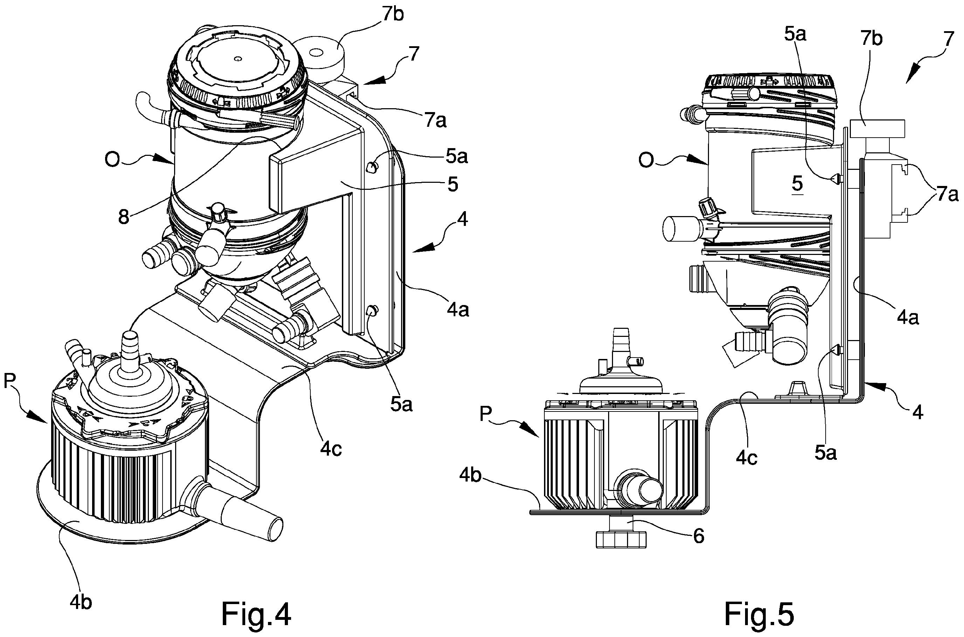

Figure 4 is an axonometric view of the holding element of the appliance in Figure 1;
Figure 5 is a side elevation view of the holding element in Figure 4.

### Embodiments of the Invention

With particular reference to these illustrations, reference numeral 1 globally indicates an appliance for the support of biomedical devices during extracorporeal circulation, in particular for the support of an oxygenator and of the relevant pumping assembly.

The appliance 1 comprises at least one load-bearing body 2, preferably of the transportable type and positionable on a reference surface, at least one command and control unit 3 operationally connectable to at least one biomedical device to control the functionalities thereof, and at least one holding element 4 associated with the load-bearing body 2 and provided with first supporting means 5 of a first biomedical device O and with second supporting means 6 of a second biomedical device P.

More particularly, the first biomedical device O is of the type of an oxygenator and the second biomedical device P corresponds to the relevant pumping assembly, e.g. of the type of a centrifugal pump. In the embodiment shown in the figures, the centrifugal pump P, although not part of the present invention, is of the dragging or magnetic levitation type and comprises at least one hollow body inside which is housed a rotor element (impeller) adapted to transfer the venous blood coming from a patient to the oxygenator O and at least one motor adapted to control the rotor element at least in rotation.

The command and control unit 3 is connectable to the centrifugal pump P, comprises at least one microprocessor which is programmed to control the relevant motor and is suitably provided with a plurality of drives 3a that can be operated by one operator as well as with at least one display 3b for displaying the parameters that can be controlled by means of the drives 3a. More in detail, the drives 3a allow controlling at least the number of revolutions of the rotor element and the flow rate delivered.

Appropriately, the load-bearing body 2 has a front portion 2a, intended to be facing the patient, a rear portion 2b, opposite the front portion 2a and accessible by an operator to actuate the drives 3a, and two lateral portions 2c interposed between the front portion 2a and the rear portion 2b.

According to the invention, the first supporting means 5 and the second supporting means 6 are locked to each other and the holding element 4 comprises removable hooking means 7 to the load-bearing body 2.

The holding element 4 is advantageously made in a single body piece.

In the preferred embodiment shown in the figures, the holding element 4 has a substantially slab-like conformation, i.e. the thickness thereof is significantly lower than the other dimensions.

Preferably, the holding element 4 has at least a first holding wall 4a with which the first supporting means 5 are associated and at least a second holding wall 4b with which the second supporting means 6 are associated, where the first and second holding walls 4a and 4b are arranged transversely to each other.

More in detail, in use, i.e. during the use of the appliance 1 when extracorporeal circulation is applied to a patient, the first holding wall 4a is substantially arranged (i.e. less the machining tolerances and/or the positioning on a non-planar reference surface) vertically and the second holding wall 4b is substantially arranged (i.e. less the machining tolerances and/or the positioning on a non-planar reference surface) horizontally.

The first supporting means 5 are advantageously arranged at a greater height with respect to the second supporting means 6.

The result, therefore, is that, in use, the oxygenator O is arranged on top of the relevant pumping assembly P, thus facilitating, from a fluid-dynamic point of view, the transfer of blood from the pumping assembly P to the oxygenator O. Appropriately, the holding element 4 comprises at least one intermediate wall 4c adjacent to the first holding wall 4a, arranged substantially transverse thereto and adapted to be arranged, in use, below the oxygenator O.

More in detail, the intermediate wall 4c is arranged substantially (i.e. less the machining tolerances) parallel to the second holding wall 4b and at a greater height with respect thereto.

In the embodiment shown in the figures, the first holding wall 4a is arranged substantially perpendicular to the second holding wall 4b and to the intermediate wall 4c.

The second holding wall 4b and the intermediate wall 4c define in practice therefore two substantially parallel and staggered planes.

Preferably, the first supporting means 5 comprise at least one housing seat 8 associable with the oxygenator O and configured to keep the oxygenator itself in suspension on the first holding wall 4a. More particularly, the housing seat 8 has a curvilinear extension in such a way as to at least partly wind the body of the oxygenator O. The housing seat 8 is configured so as to receive the oxygenator O by interlocking and/or defines at least one abutment surface on which the oxygenator itself rests.

Advantageously, the first supporting means 5 are associated with the holding element 4 in a removable manner. More specifically, the first supporting means 5 comprise removable fixing means 5a, e.g. of the type of one or more interlocking elements, to the holding element 4.

Appropriately, the first supporting means 5 are of the disposable type and are intended to be applied to the oxygenator O prior to the sterilization thereof.

The second supporting means 6 are instead arranged at the second holding wall 4b and are configured to keep the centrifugal pump P in position on the second holding wall itself.

More in detail, the second supporting means 6 can be of the type of a threaded member (as in the embodiment shown in the figures) which passes through the second holding wall 4b and which is adapted to engage with the containment body of the motor of the centrifugal pump P, or they can be of the interlocking type, slide type or other type known to the expert in the field.

Advantageously, the hooking means 7 are movable from at least one engagement configuration to a release configuration with the load-bearing body 2. In the preferred embodiment shown in the figures, the hooking means 7 comprise two hooking elements 7a movable in the direction of mutual close/away movement. More particularly, in the engagement configuration, these hooking elements 7a are close to each other and are movable in the direction of mutual away movement, counteracting elastic means (not visible in detail in the figures), in order to reach the release configuration.

Appropriately, the hooking means 7 comprise at least one activation element 7b which can be activated by an operator to bring the hooking elements 7a from the engagement configuration to the release configuration.

In turn, the appliance 1 comprises at least one gripping element 9 associated with the load-bearing body 2 and with which the hooking means 7 are adapted to engage in a removable manner.

In the preferred embodiment shown in the figures, the gripping element 9 is of the type of an element with an elongated shape and protruding from the load-bearing body 2 so as to define a profile engageable by the hooking elements 7a in the engagement configuration. Alternatively, the gripping element 9 can be recessed with respect to the load-bearing body 2.

Conveniently, the gripping element 9 is of the joined type, i.e. its conformation and its dimensions are made according to codified and standardized regulations. In particular, the gripping element 9 corresponds to the profile also found on stretchers used in hospitals.

Advantageously, the appliance 1 comprises at least two gripping elements 9 which are separate from each other, so as to allow positioning the holding element 4 on the load-bearing body 2 in at least two different positions that can be selected by the operator according to the specific needs.

More in detail, at least one of the gripping elements 9 is associated with the front portion 2a of the load-bearing body 2, while another gripping element 9 is associated with at least one of the lateral portions 2c of the load-bearing body itself. This way the holding element 4 is positionable on the load-bearing body 2, at the operator's will, in at least two positions which are substantially orthogonal to each other.

Preferably, the appliance 1 comprises first protection means 10 of the first biomedical device, i.e. the oxygenator O. More particularly, the first protection means 10 are associated with the load-bearing body 2 and are shaped so as to surround the oxygenator O. In the preferred embodiment shown in the figures, the first protection means 10 are e.g. of the type of a curved bar. Appropriately, the first protection means 10 are movable from a work position, wherein they are arranged to surround the oxygenator O, and a home position, wherein they are displaced with respect to the work position so as to facilitate the application or removal of the holding element 4 to/from the load-bearing body 2. In the embodiment shown in the figures, the first protection means 10 are hinged to the load-bearing body 2 at its front portion 2a and rotate upwards to displace from the work position to the home position. More specifically, the first protection means 10 are associated with the load-bearing body 2 in a removable manner so as to reduce the overall dimensions of the appliance 1.

Advantageously, the appliance 1 also comprises second protection means 11 of the second biomedical device, i.e. of the pumping assembly P. More particularly, the second protection means 11 are associated with the load-bearing body 2 and are shaped so as to surround the pumping assembly P. Appropriately, the second protection means 11 are movable between a work position, wherein they are arranged to surround the pumping assembly P, and a home position, wherein they are displaced from the work position in such a way as to reduce the overall dimensions of the appliance 1. In the embodiment shown in the figures, the second protection means 11 are hinged to the load-bearing body 2 at its front portion 2a and rotate upwards to move from the work position to the home position. In particular, the second protection means 11 are of the type of a curved bar shaped so as to resume, in the home position, the profile of the front portion 2a on which it rests.

The operation of the present invention is as follows.

Depending on the specific needs, i.e. on the position of the patient and of the other devices that make up the appliance used for extracorporeal circulation, the operator positions the holding element 4 at the front portion 2a or the lateral portion 2c of the load-bearing body 2.

In particular, the operator operates the activation element 7b to bring the hooking elements 7a to the release configuration and, after they have been positioned at the gripping element 9 of interest, releases the activation element itself so as to allow it to be engaged with the gripping element 9.

This way the holding element 4 is locked together with the load-bearing body 2. The operator then positions the oxygenator O and the pumping assembly P on the holding element 4 by means of the first and second supporting means 5 and 6 respectively.

As mentioned above, the oxygenator O is preferably associated with the first supporting means 5 before the sterilization thereof. The first supporting means 5 are, therefore, of the disposable type.

More in detail, the oxygenator O is fitted inside the housing seat 8, while the centrifugal pump P is positioned at the second holding wall 4b and blocked with respect thereto by means of the threaded member 6.

After the various devices have been connected to each other and have been connected to the patient, it is possible to start the extracorporeal circulation by monitoring and possibly modifying some parameters of the centrifugal pump P, such as the number of revolutions and the flow rate delivered, by means of the command and control unit 3.

In case the patient is placed on a stretcher, whether in hospital or out of hospital, it is possible, always operating on the hooking means 7, to release the holding element 4 from the load-bearing body 2. In particular, the operator can operate again on the activation element 7b to bring the hooking elements 7a to the release configuration and thus remove the holding element 4 from the load-bearing body 2. The holding element 4 can then be applied to the stretcher's structure, always using the hooking means 7.

Appropriately, the holding element 4 is arranged at a lower height than the patient's one in order to optimize the fluid dynamics of blood circulation.

It has in practice been ascertained that the described invention achieves the intended objects and in particular it is emphasized that it allows optimally positioning the oxygenator and the relevant pumping assembly during the extracorporeal circulation applied to a patient.

In particular, the shape of the holding element makes it possible to move both the oxygenator and the centrifugal pump at the same time, keeping them in the same mutual position.

In addition, the hooking means make it possible both to vary the position of the holding element on the load-bearing body and to apply it to a hospital stretcher, thus making it flexible and practical to use.

## Claims

1. Appliance (1) for the support of biomedical devices during extracorporeal circulation, comprising at least one load-bearing body (2), at least one command and control unit (3) connectable to at least one biomedical device, at least one holding element (4) provided with at least first supporting means (5) of a first biomedical device (O) and with second supporting means (6) of a second biomedical device (P), wherein said first supporting means (5) and said second supporting means (6) are locked to each other and wherein said holding element (4) comprises removable hooking means (7) to said load-bearing body (2), said holding element (4) having at least a first holding wall (4a) with which said first supporting means (5) are associated and having at least a second holding wall (4b) with which said second supporting means (6) are associated, said first and second holding walls (4a, 4b) being arranged transversely to each other, **characterized by** the fact that said holding element (4) comprises at least one intermediate wall (4c) adjacent to said first holding wall (4a) and arranged substantially transverse thereto, said intermediate wall (4c) being adapted to be arranged, in use, below the first biomedical device (O).

2. Appliance (1) according to claim 1, **characterized by** the fact that said holding element (4) is made in a single body piece.

3. Appliance (1) according to one or more of the preceding claims, **characterized by** the fact that said first and second holding walls (4a, 4b) are arranged, in use, vertically and horizontally, respectively.

4. Appliance (1) according to one or more of the preceding claims, **characterized by** the fact that said intermediate wall (4c) is arranged substantially parallel to said second holding wall (4b) and at a greater height with respect thereto.

5. Appliance (1) according to one or more of the preceding claims, **characterized by** the fact that said first supporting means (5) comprise at least one housing seat (8) of the first biomedical device (O) and configured to keep the latter in suspension on said first holding wall (4a).

6. Appliance (1) according to one or more of the preceding claims, **characterized by** the fact that said first supporting means (5) are associated in a removable manner with said holding element (4).

7. Appliance (1) according to claim 6, **characterized by** the fact that said first supporting means (5) comprise removable fixing means (X) to said holding element (4).

8. Appliance (1) according to one or more of the preceding claims, **characterized by** the fact that said second supporting means (6) are configured to keep the second biomedical device (P) in position on said second holding wall (4b).

9. Appliance (1) according to one or more of the preceding claims, **characterized by** the fact that said hooking means are movable from at least one engagement configuration to a release configuration with said load-bearing body.

10. Appliance (1) according to one or more of the preceding claims, **characterized by** the fact that it comprises at least one gripping element (9) associated with said load-bearing body (2) and by the fact that said hooking means (7) are adapted to engage in a removable manner with said gripping element (9).

11. Appliance (1) according to claim 10, **characterized by** the fact that it comprises at least two of said gripping elements (9) which are separate from each other.

12. Appliance (1) according to claim 11, **characterized by** the fact that said load-bearing body (2) comprises at least one front portion (2a) and at least two lateral portions (2c) arranged transversely with respect to said front portion (2a), said gripping elements (9) being associated with at least said front portion (2a) and with at least one of said lateral portions (2c).

13. Appliance (1) according to one or more of the preceding claims, **characterized by** the fact that it comprises at least one of first protection means (10) of the first biomedical device (O) and second protection means (11) of the second biomedical device (P) associated with said load-bearing body (2).

14. Appliance (1) according to claim 13, **characterized by** the fact that at least one of said first protection means (10) and said second protection means (11) is movable with respect to said load-bearing body (2) from a work position, wherein it is arranged to surround the relevant biomedical device (O, P), and a home position, wherein it is displaced with respect to the work position.

15. Appliance (1) according to claim 13 or 14, **characterized by** the fact that at least one of said first protection means (10) and said second protection means (11) is hinged to said load-bearing body (2) at said front portion (2a).

## Patentansprüche

1. Vorrichtung (1) zur Halterung von biomedizinischen Geräten während des extrakorporalen Kreislaufs, umfassend mindestens einen lasttragenden Körper (2), mindestens eine Befehls- und Steuereinheit (3), die mit mindestens einem biomedizinischen Gerät verbindbar ist, mindestens ein Halteelement (4), das mit mindestens ersten Stützmitteln (5) eines ersten biomedizinischen Gerätes (O) und mit zweiten Stützmitteln (6) eines zweiten biomedizinischen Gerätes (P) versehen ist, wobei die ersten Stützmittel (5) und die zweiten Stützmittel (6) miteinander verriegelt sind und wobei das Halteelement (4) abnehmbare Hakenmittel (7) zu dem lasttragenden Körper (2) aufweist, wobei das Halteelement (4) mindestens eine erste Haltewand (4a) aufweist, der die ersten Stützmittel (5) zugeordnet sind, und mindestens eine zweite Haltewand (4b) aufweist, der die zweiten Stützmittel (6) zugeordnet sind, wobei die erste und die zweite Haltewand (4a, 4b) quer zueinander angeordnet sind,
**dadurch gekennzeichnet, dass** das Halteelement (4) mindestens eine Zwischenwand (4c) aufweist, die an die erste Haltewand (4a) angrenzt und im Wesentlichen quer dazu angeordnet ist, wobei die Zwischenwand (4c) dazu ausgebildet ist, im Gebrauch unterhalb des ersten biomedizinischen Geräts (O) angeordnet zu sein.

2. Vorrichtung (1) nach Anspruch 1, **dadurch gekennzeichnet, dass** das Halteelement (4) in einem einzigen Teil hergestellt ist.

3. Vorrichtung (1) nach einem oder mehreren der vorhergehenden Ansprüche, **dadurch gekennzeichnet, dass** die erste und zweite Haltewand (4a, 4b) im Gebrauch vertikal bzw. horizontal angeordnet sind.

4. Vorrichtung (1) nach einem oder mehreren der vorhergehenden Ansprüche, **dadurch gekennzeichnet, dass** die Zwischenwand (4c) im Wesentlichen parallel zu der zweiten Haltewand (4b) und in einer größeren Höhe in Bezug auf diese angeordnet ist.

5. Vorrichtung (1) nach einem oder mehreren der vorhergehenden Ansprüche, **dadurch gekennzeichnet, dass** die ersten Stützmittel (5) mindestens einen Aufnahmesitz (8) des ersten biomedizinischen Geräts (O) aufweisen und dazu ausgebildet sind, letzteres an der ersten Haltewand (4a) in Aufhängung zu halten.

6. Vorrichtung (1) nach einem oder mehreren der vorhergehenden Ansprüche, **dadurch gekennzeichnet, dass** die ersten Stützmittel (5) dem Halteelement (4) in lösbarer Weise zugeordnet sind.

7. Vorrichtung (1) nach Anspruch 6, **dadurch gekennzeichnet, dass** die ersten Stützmittel (5) abnehmbare Befestigungsmittel (X) zu dem Halteelement (4) aufweisen.

8. Vorrichtung (1) nach einem oder mehreren der vorhergehenden Ansprüche, **dadurch gekennzeichnet, dass** die zweiten Stützmittel (6) dazu ausgebildet sind, das zweite biomedizinische Gerät (P) an der zweiten Haltewand (4b) in Position zu halten.

9. Vorrichtung (1) nach einem oder mehreren der vorhergehenden Ansprüche, **dadurch gekennzeichnet, dass** die Hakenmittel von mindestens einer Eingriffskonfiguration in eine Freigabekonfiguration mit dem lasttragenden Körper bewegbar sind.

10. Vorrichtung (1) nach einem oder mehreren der vorhergehenden Ansprüche, **dadurch gekennzeichnet, dass** sie mindestens ein dem lasttragenden Körper (2) zugeordnetes Greifelement (9) aufweist und dass die Hakenmittel (7) ausgebildet sind, um lösbar mit dem Greifelement (9) in Eingriff zu kommen.

11. Vorrichtung (1) nach Anspruch 10, **dadurch gekennzeichnet, dass** sie mindestens zwei der Greifelemente (9) aufweist, die voneinander getrennt sind.

12. Vorrichtung (1) nach Anspruch 11, **dadurch gekennzeichnet, dass** der lasttragende Körper (2) mindestens einen vorderen Abschnitt (2a) und mindestens zwei seitliche Abschnitte (2c) aufweist, die quer zum vorderen Abschnitt (2a) angeordnet sind, wobei die Greifelemente (9) mindestens dem vorderen Abschnitt (2a) und mindestens einem der seitlichen Abschnitte (2c) zugeordnet sind.

13. Vorrichtung (1) nach einem oder mehreren der vorhergehenden Ansprüche, **dadurch gekennzeichnet, dass** sie mindestens erste Schutzmittel (10) des ersten biomedizinischen Geräts (O) und/oder zweite Schutzmittel (11) des zweiten biomedizinischen Geräts (P) aufweist, die dem lasttragenden Körper (2) zugeordnet sind.

14. Vorrichtung (1) nach Anspruch 13, **dadurch gekennzeichnet, dass** mindestens eines der ersten Schutzmittel (10) und der zweiten Schutzmittel (11) in Bezug auf den lasttragenden Körper (2) aus einer Arbeitsposition, in der es so angeordnet ist, dass es das betreffende biomedizinische Gerät (O, P) umgibt, und einer Grundposition, in der es in Bezug auf die Arbeitsposition verlagert ist, beweglich ist.

15. Vorrichtung (1) nach Anspruch 13 oder 14, **dadurch gekennzeichnet, dass** mindestens eines der ersten Schutzmittel (10) und der zweiten Schutzmittel (11) an dem lasttragenden Körper (2) an dem vorderen Schutz (2a) angelenkt ist.

## Revendications

1. - Appareil (1) pour le support de dispositifs biomédicaux pendant une circulation extracorporelle, comprenant au moins un corps porteur (2), au moins une unité de commande et de contrôle (3) apte à être reliée à au moins un dispositif biomédical, au moins un élément de maintien (4) comportant au moins des premiers moyens de support (5) d'un premier dispositif biomédical (O) et des seconds moyens de support (6) d'un second dispositif biomédical (P), dans lequel lesdits premiers moyens de support (5) et lesdits seconds moyens de support (6) sont verrouillés les uns aux autres et dans lequel ledit élément de maintien (4) comprend des moyens d'accrochage amovibles (7) audit corps porteur (2), ledit élément de maintien (4) ayant au moins une première paroi de maintien (4a) à laquelle sont associés lesdits premiers moyens de support (5) et ayant au moins une seconde paroi de maintien (4b) à laquelle sont associés lesdits seconds moyens de support (6), lesdites première et seconde parois de maintien (4a, 4b) étant disposées transversalement l'une par rapport à l'autre,
**caractérisé par le fait que** ledit élément de maintien (4) comprend au moins une paroi intermédiaire (4c) adjacente à ladite première paroi de maintien (4a) et disposée sensiblement transversalement à celle-ci, ladite paroi intermédiaire (4c) étant apte à être disposée, lors de l'utilisation, au-dessous du premier dispositif biomédical (O).

2. - Appareil (1) selon la revendication 1, **caractérisé par le fait que** ledit élément de maintien (4) est réalisé en une seule pièce de corps.

3. - Appareil (1) selon une ou plusieurs des revendications précédentes, **caractérisé par le fait que** lesdites première et seconde parois de maintien (4a, 4b) sont disposées, lors de l'utilisation, respectivement verticalement et horizontalement.

4. - Appareil (1) selon une ou plusieurs des revendications précédentes, **caractérisé par le fait que** ladite paroi intermédiaire (4c) est disposée sensiblement parallèlement à ladite seconde paroi de maintien (4b) et à une plus grande hauteur par rapport à celle-ci.

5. - Appareil (1) selon une ou plusieurs des revendications précédentes, **caractérisé par le fait que** lesdits premiers moyens de support (5) comprennent au moins un siège de boîtier (8) du premier dispositif biomédical (O) et configuré pour maintenir ce dernier en suspension sur ladite première paroi de maintien (4a).

6. - Appareil (1) selon une ou plusieurs des revendications précédentes, **caractérisé par le fait que** lesdits premiers moyens de support (5) sont associés de manière amovible audit élément de maintien (4).

7. - Appareil (1) selon la revendication 6, **caractérisé par le fait que** lesdits premiers moyens de support (5) comprennent des moyens de fixation amovibles (X) audit élément de maintien (4).

8. - Appareil (1) selon une ou plusieurs des revendications précédentes, **caractérisé par le fait que** lesdits seconds moyens de support (6) sont configurés pour maintenir le second dispositif biomédical (P) en position sur ladite seconde paroi de maintien (4b).

9. - Appareil (1) selon une ou plusieurs des revendications précédentes, **caractérisé par le fait que** lesdits moyens d'accrochage sont déplaçables d'au moins une configuration d'engagement à une configuration de libération avec ledit corps porteur.

10. - Appareil (1) selon une ou plusieurs des revendications précédentes, **caractérisé par le fait qu'**il comprend au moins un élément de préhension (9) associé audit corps porteur (2) et **par le fait que** lesdits moyens d'accrochage (7) sont aptes à s'engager de manière amovible avec ledit élément de préhension (9).

11. - Appareil (1) selon la revendication 10, **caractérisé par le fait qu'**il comprend au moins deux desdits éléments de préhension (9) qui sont séparés l'un de l'autre.

12. - Appareil (1) selon la revendication 11, **caractérisé par le fait que** ledit corps porteur (2) comprend au moins une partie avant (2a) et au moins deux parties latérales (2c) disposées transversalement par rapport à ladite partie avant (2a), lesdits éléments de préhension (9) étant associés à au moins ladite partie avant (2a) et à au moins l'une desdites parties latérales (2c) .

13. - Appareil (1) selon une ou plusieurs des revendications précédentes, **caractérisé par le fait qu'**il comprend au moins l'un de premiers moyens de protection (10) du premier dispositif biomédical (O) et de seconds moyens de protection (11) du second dispositif biomédical (P) associés audit corps porteur (2).

14. - Appareil (1) selon la revendication 13, **caractérisé par le fait qu'**au moins l'un desdits premiers moyens de protection (10) et desdits seconds moyens de protection (11) est déplaçable par rapport audit corps porteur (2) à partir d'une position de travail, dans laquelle il est disposé pour entourer le dispositif biomédical concerné (O, P), à une position de repos, dans laquelle il est déplacé par rapport à la position de travail.

15. - Appareil (1) selon l'une des revendications 13 ou 14, **caractérisé par le fait qu'**au moins l'un desdits premiers moyens de protection (10) et desdits seconds moyens de protection (11) est articulé audit corps porteur (2) à ladite partie avant (2a).
